# EUROPÄISCHE PATENTSCHRIFT

(11) **EP 0 278 914 B1**
(45) Veröffentlichungstag und Bekanntmachung des Hinweises auf die Patenterteilung: **22.09.1993**
(21) Anmeldenummer: 88810074.0
(22) Anmeldetag: 08.02.1988
(51) Int. Cl.: C07C 67/03, C07C 69/40, C07C 69/78, C07C 69/24, C07C 69/34, C07C 69/76

(54) **Verfahren zur Herstellung von tert.-Alkylestern der Bernsteinsäure**
Process for the preparation of tert.-alkyl esters of succinic acid
Procédé de préparation d'esters tertio-alkyliques de l'acide succinique

(30) Priorität: 13.02.1987 CH 534/87
(43) Veröffentlichungstag der Anmeldung: 17.08.1988
(73) Patentinhaber: CIBA-GEIGY AG, 4002 Basel (CH)
(72) Erfinder: Frei, Urs, CH-3211 Liebistorf (CH); Kirchmayr, Rudolf, Dr., CH-1723 Marly (CH)

(56) Entgegenhaltungen:
- DE-B- 1 124 950
- FR-A- 2 013 254

## Beschreibung

Die vorliegende Erfindung betrifft ein Verfahren zur Herstellung von tert.-Alkylestern der Bernsteinsäure durch katalytische Umesterung von Methyl- oder Ethylestern mit tert.-Alkylalkoholen.

Katalytische Umesterungen von primären Niederalkylestern höherer Carbonsäuren mit tertiären Alkoholen zu tertiären Estern, in Analogie zu den allgemein bekannten Methoden Zur Herstellung von längerkettigen primären und sekundären Alkylestern, sind in DE-A-1 124 950 beschrieben.

In Synthesis 1972, 49 beschreiben D.S. Wulfman et al. eine Teilumesterung von 7,7-Dimethoxycarbonylnorcaran zu 7-exo-tert.-Butoxycarbonyl-7-endo-methoxycarbonylnorcaran durch Umsetzung mit Kalium-t.-butylat im Molverhältnis 1:1 in t.-Butylalkohol in Gegenwart von Molekularsieben. Diese Methode ist nicht allgemein und insbesondere nicht für die technische Herstellung von tertiären Estern anwendbar, da bestimmte Carbonsäureester in Gegenwart von solch grossen Mengen an Basen (1:1 Mol) unerwünschte Nebenreaktionen eingehen. Die unumgängliche Entfernung der so entstandenen störenden Nebenprodukte ist zeit-, arbeits-und energieaufwendig.

Es ist nun gefunden worden, dass durch Umsetzung von Methyl- oder Ethylestern der Bernsteinsäure mit tert.-Alkylalkoholen, in Gegenwart katalytischer Mengen Lithium, Lithiumamid oder Natrium, tert.-Alkylester überraschenderweise mit ausgezeichneten Ausbeuten und ohne Bildung störender Nebenprodukte erhalten werden können.

Die vorliegende Erfindung betrifft demnach ein Verfahren zur Herstellung von tert.-Alkylestern der Formel I 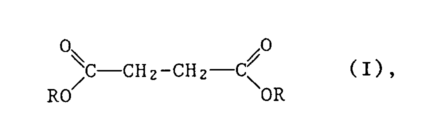
worin
R tert.-Butyl oder tert.-Pentyl bedeutet.
durch Umsetzung eines Esters der Formel II 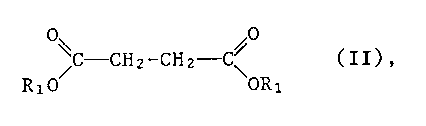
worin R₁ Methyl oder Ethyl ist, mit 4-20, bevorzugt 8-12 Molen eines Alkohols der Formel ROH, worin R die oben angegebene Bedeutung hat, in Gegenwart katalytischer Mengen eines Katalysators ausgewählt aus der Gruppe bestehend aus Lithium, Lithiumamid oder Natrium unter gleichzeitigem Abdestillieren des sich bildenden Methanols oder Ethanols.

Bevorzugt geht man von einem Ester der Formel II aus, worin R₁ Methyl ist.

Der Ester der Formel II wird zweckmässig bei Raumtemperatur mit der erforderlichen Menge tert.-Alkohol der Formel ROH vermischt. Danach wird der Katalysator in Mengen von 8-30, bevorzugt 10-15 Mol %, bezogen auf den Ester der Formel II, zugegeben. Das Reaktionsgemisch wird am Rückfluss erhitzt, wobei der entstehende primäre Alkohol der Formel R₁OH, teilweise mit tert.-Alkohol der Formel ROH vermischt, über eine Fraktionierkolonne abdestilliert wird. Zu diesem Zwecke sind Vigreux- und insbesondere Füllkörperkolonnen sehr gut geeignet. Die Menge an abdestilliertem Alkoholgemisch wird zweckmässig alle drei Stunden mit neuem tert.-Alkohol ersetzt. Nach 16-30, bevorzugt 20-26 Stunden erhält man die gewünschten tert.-Alkylester mit ausgezeichneten Ausbeuten.

Bei den Estern der Formel II und den Alkoholen der Formel ROH handelt es sich um bekannte Substanzen.

Die folgenden Beispiele erläutern die Erfindung, ohne sie einzuschränken.
Beispiel 1: 146 g Bernsteinsäuredimethylester (1 Mol) werden bei Raumtemperatur in 1 Liter tert.-Amylalkohol abs. (11 Mol) vorgelegt. Danach werden 0,7 g Lithium (0,1 Mol) zugegeben. Das Reaktionsgemisch wird am Rückfluss bei ca. 100°C gekocht, und das gebildete Methanol, teilweise vermischt mit tert.-Amylalkohol, wird fortlaufend über eine Fraktionierkolonne (Füllkörperkolonne) bei 86-87°C abdestilliert. Die Menge des abdestillierten Alkoholgemisches wird alle drei Stunden mit neuem tert.-Alkohol ergänzt (insgesamt ca. 350 ml). Nach 22 Stunden wird der Alkohol vollständig abdestilliert und der ölige Rückstand mit Wasser/Cyclohexan zur Entfernung des Lithiumsalzes gewaschen. Die organische Phase wird abgetrennt, eingedampft und im Vakuum destilliert (65°C bei 10 Pa). Man erhält 200 g (77 % d.Th.) an reinem Bernsteinsäure-di-tert.-pentylester.

| Analyse für C₁₄H₂₆O₄ | | |
|---|---|---|
| | C | H |
| Ber. | 65,09 | 10,14 |
| Gef. | 64,9 | 10,1 |

Beispiele 2-4: Verfährt man wie in Beispiel 1, ausgehend von 1 Mol Ester der Formel 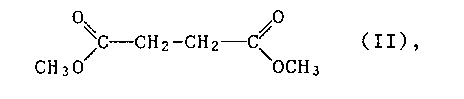 und einem tert.-Alkohol ROH, in Gegenwart von 0,1 Mol Katalysator, wobei die Bedeutung von R, der Katalysator, die Menge an tert.-Alkohol und die Reaktionsdauer in der nachfolgenden Tabelle angegeben sind, so erhält man die entsprechenden tert.-Ester der Formel I.

| Bsp. | R | Menge tert.-Alkohol | Katalysator | Reaktionsdauer in Std. |
|---|---|---|---|---|
| 2 | tert.-Pentyl | 11 Mol | LiNH₂ | 22 |
| 3 | tert.-Pentyl | 11 Mol | Natrium | 22 |
| 4 | tert.-Pentyl | 11 Mol | Lithium | 22 |

Beispiel 5: Verfährt man wie in Beispiel 1 mit der einzigen Ausnahme, dass man von Bernsteinsäurediethylester ausgeht, so erhält man den Bernsteinsäure-di-tert.-pentylester mit ebenso guter Ausbeute und Reinheit wie in Beispiel 1.

## Patentansprüche

1. Verfahren zur Herstellung von tert.-Alkylestern der Formel 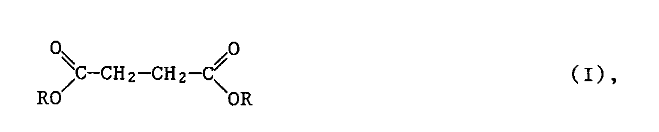 worin R tert.-Butyl oder tert.-Pentyl bedeutet,
durch Umsetzung eines Esters der Formel 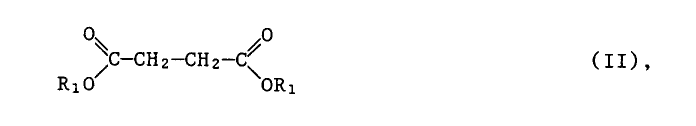 worin R₁ Methyl oder Ethyl ist, mit 4-20 Molen eines Alkohols der Formel ROH, worin R die oben angegebene Bedeutung hat, in Gegenwart katalytischer Mengen eines Katalysators ausgewählt aus der Gruppe bestehend aus Lithium, Lithiumamid oder Natrium, unter gleichzeitigem Abdestillieren des sich bildenden Methanols oder Ethanols.

2. Verfahren gemäss Anspruch 1, dadurch gekennzeichnet, dass man von einem Ester der Formel II ausgeht, worin R₁ Methyl ist.

3. Verfahren gemäss Anspruch 1, dadurch gekennzeichnet, dass man den Katalysator in einer Menge von 8-30 Mol %, bezogen auf den Ester der Formel II einsetzt.

## Claims

1. A process for the preparation of a tert-alkyl ester of formula 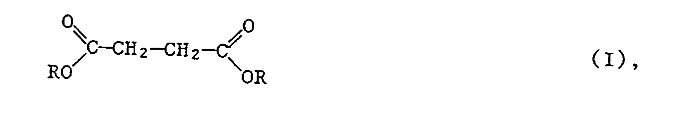 wherein R is tert-butyl or tert-pentyl,
by reaction of an ester of formula 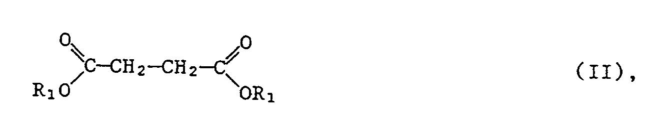 wherein R₁ is methyl or ethyl, with 4 to 20 mol of an alcohol of formula ROH, wherein R is as defined above, in the presence of a catlaytic amount of a catalyst selected from the group consisting of lithium, lithium amide or sodium, with simultaneous removal of methanol or ethanol by distillation.

2. A process according to claim 1, wherein the starting material is an ester of formula II, wherein R₁ is methyl.

3. A process according to claim 1, wherein the catalyst is used in an amount of 8-30 % molar, based on the ester of formula II.

## Revendications

1. Procédé de préparation d'esters tert.-alkyliques de formule 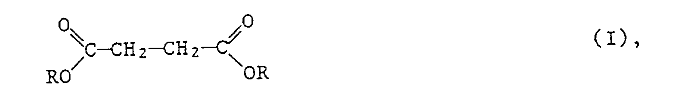 R désignant un groupe tert. -butyle où tert.-pentyle, par réaction d'un ester de formule 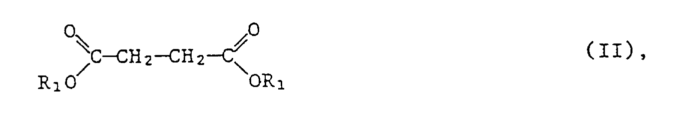 R₁ désignant le groupe méthyle ou éthyle, avec de 4 à 20 moles d'un alcool ROH en présence d'une proportion catalytique d'un catalyseur pris parmi le lithium, l'amidure de lithium et le sodium, tout en éliminant par distillation le méthanol ou l'éthanol formés.

2. Procédé selon la revendication 1, caractérisé en ce que dans l'ester de formule (II) R₁ est le groupe méthyle.

3. Procédé selon la revendication 1, caractérisé en ce que l'on opère avec 8 à 30 Mol% du catalyseur par rapport à l'ester de formule (II).
